# EUROPEAN PATENT APPLICATION

(11) **EP 2 767 173 A1**
(43) Date of publication of application: **20.08.2014**
(21) Application number: 14168113.0
(22) Date of filing: 27.04.2011
(51) Int. Cl.: A23L 1/29, A61K 31/702, A61P 1/12

(54) **Use of human milk oligosaccharides in infant nutrition**

(30) Priority: 27.04.2010 WO PCT/NL2010/050240; 11.05.2010 EP 10162530
(62) Divisional of application: 11720600.3
(71) Applicant: N.V. Nutricia, 2712 HM Zoetermeer (NL)
(72) Inventor: Stahl, Bernd, 3584 CT Utrecht (NL)
(74) Representative: Nederlandsch Octrooibureau

(57) **Abstract**

A method for feeding an infant is provided, wherein the diet of the infant is supplemented with 2-fucosylated oligosaccharides, dependent on the blood group of the infant, preferably in combination with the blood group of the infant's mother.

## Description

### FIELD OF THE INVENTION

The present invention is in the field of tailor-made infant and toddler nutrition, depending on the blood group or blood type of the mother and of the infant.

### BACKGROUND OF THE INVENTION

The Lewis blood typing system distinguishes the antigens Lewis a and Lewis b (Le a and Le b) and Lewis x and Lewis y (Le x and Le y). The core structure of Lewis x and y are built up differently compared to Lewis a and b, but in terms of carbohydrate units Le a corresponds to Le x and Le b corresponds to Le y. These antigens are soluble antigens which may be present in body fluids including human milk. There are three phenotypes: Le(a-/b-); Le(a+/b-); and Le(a-/b+). The presence of fucosyltransferase converts the Lewis a antigen to Lewis b. The same enzyme also converts Lewis x to Lewis y. Individuals with Lewis a antigens are usually non-secretors, and the presence of the Lewis b antigen makes a person a secretor. Lewis negative (Le a-, Le b-) can be either secretors or non-secretors.

The basis for the phenotypic difference between the secretor and non-secretor subpopulations stems from genetic polymorphisms resulting in the expression of a specific functional alpha-1,2-fucosyltransferase in case of secretors whereas non-secretors do not express this functional alpha-1,2-fucosyltransferase. Accordingly, soluble 2-fucosylated group antigens are not synthesized and therefore not secreted into biological fluids of non-secretors. However, their epithelia and other surfaces may contain these structures serving also as adhesion points for microbiota including pathogens. Thus a non secretor mother is lacking substances in her milk which may or may not be needed based on the respective receptor structures of the baby as recipient of the milk.

WO 2009/033011 concerns a diagnostic method for identifying an individual at risk for necrotizing enterocolitis and related disorders by measuring the level of secretor antigens, in particular H-antigens and Lewis antigens, in saliva.

Several biological functions of human milk oligosaccharides have been postulated so far. Human milk oligosaccharides are considered to protect breast-fed infants from pathogenic bacteria, viruses, toxins, protozoa and fungi by acting as soluble receptor analogues that prevent the interaction of pathogens with epithelial cells in the initial step of infections. Furthermore, such oligosaccharides may act as prebiotics, hence to promote growth of *Bifidobacterium bifidum* while thereby suppressing growth of undesirable bacteria.

Depending on the blood group, variations and different patterns of oligosaccharides in human milk occur (Thurl et al. 1997, Glycoconjugate J. 14:795-799). More detailed facts about these important human milk components will be helpful for the development of better adapted milk formulas.

WO 98/43494 concerns the analysis of a large number of human milk samples to determine appropriate average levels of nine important milk oligosaccharides and to arrive at the preparation of a synthetic or manufactured / artificial infant formulation containing these oligosaccharides near the naturally occurring levels found in human breast milk.

### SUMMARY OF THE INVENTION

It has now been found that infant nutrition can be more properly adapted by taking the blood group of the infant into account. The present inventors found that non-secretor infants have limited or no benefit of their nutrition being supplemented with human milk oligosaccharides for which there are no genetically based surface analogues (epitopes) expressed. On the other hand however, secretor infants do have the corresponding epitopes in particular in their secretions. It is important that the nutrition of these secretor infants contain all the soluble counterparts for these receptors, in particular because these receptors are for example targeted by pathogens like E.coli. Thus, if not adequately fed, secretor infants have an increased risk of bacterial infection.

Furthermore the secretor type milks offer more specific substrates for the beneficial microbiota like bifidus bacteria specialised on the fermentation of theses compounds from epithelia and in solution.

Consequently, the present invention thus provides tailor-made or individualised infant nutrition, specifically with regard to the blood group of the infant and advantageously taking the blood group of the mother into account.

### DETAILED DESCRIPTION

Secreting infants are characterized as having Lewis blood type Le(a-/b+) or Le(a-/b-) according to the Lewis blood typing system. For sake of clarity it is noted that secreting infants having Lewis blood type Le(a-/b-) have active secretor enzyme (FucT II).

In one embodiment secreting infants are characterized as having blood group A, B, or AB according to the ABO blood group system

Non-secreting mothers are characterized by having Lewis blood type Le(a+/b-) according to the Lewis blood typing system.

In one embodiment non-secreting mothers are characterized by having blood group O of the ABO blood group system.

The present invention thus concerns a method for feeding an infant who has a) Lewis blood type Le(a-/b+) or Le(a-/b-) according to the Lewis blood typing system and/or b) Lewis blood type Le(x-/y+) or Le(x-/y-) according to the Lewis blood typing system, said method comprising administering a nutritional composition comprising 2-fucosylated oligosaccharides.

In one embodiment the present invention concerns a method for feeding an infant who has blood group A, B, or AB according to the ABO blood group system, said method comprising administering a nutritional composition comprising 2-fucosylated oligosaccharides.

In other words the invention concerns the use of a composition comprising 2-fucosylated oligosaccharides for the preparation of a nutritional composition for feeding an infant, said infant having a) Lewis blood type Le(a-/b+) or Le(a-/b-) according to the Lewis blood typing system and/or b) Lewis blood type Le(x-/y+) or Le(x-/y-) according to the Lewis blood typing system.

In one embodiment the invention concerns the use of a composition comprising 2-fucosylated oligosaccharides for the preparation of a nutritional composition for feeding an infant, said infant having blood group A, B, or AB according to the ABO blood group system.

The nutritional composition may be a complete infant formula as such. In one embodiment the nutritional composition may also be a nutritional supplement or may be a supplemented human milk. In this embodiment, nutritional supplement comprises 2-fucosylated oligosaccharides or the human milk is supplemented with a composition comprising 2-fucosylated oligosaccharides. According to the present invention it is especially advantageous to supplement the human milk of a non-secreting mother.

In one embodiment, the present invention concerns the use of 2-fucosylated oligosaccharides for the preparation of a nutritional supplement for supplementing human milk for feeding an infant who has a) Lewis blood type Le(a-/b+) or Le(a-/b-) according to the Lewis blood typing system and/or b) Lewis blood type Le(x-/y+) or Le(x-/y-) according to the Lewis blood typing system.

In one embodiment the present invention concerns the use of 2-fucosylated oligosaccharides for the preparation of a nutritional supplement for supplementing human milk for feeding an infant who has blood group A, B, or AB according to the ABO blood group system.

Preferably the human milk that needs supplementation, or the human milk that is supplemented, is the human milk of a mother who has a) Lewis blood type Le(a+/b-) according to the Lewis blood typing system and/or b) Lewis blood type Le(x+/y-) according to the Lewis blood typing system.

In one embodiment the human milk that needs supplementation, or the human milk that is supplemented, is the human milk of a mother who has blood group O according to the ABO blood group system.

The invention also concerns a nutritional supplement comprising 2-fucosylated oligosaccharides for use in supplementing an infant formula or a human milk for feeding an infant, wherein the infant has a) Lewis blood type Le(a-/b+) or Le(a-/b-) according to the Lewis blood typing system and/or b) Lewis blood type Le(x-/y+) or Le(x-/y-) according to the Lewis blood typing system. Preferably the nutritional supplement is for supplementing human milk of a mother who has a) Lewis blood type Le(a+/b-) according to the Lewis blood typing system and/or b) Lewis blood type Le(x+/y-) according to the Lewis blood typing system.

In one embodiment the invention also concerns a nutritional supplement comprising 2-fucosylated oligosaccharides for use in supplementing an infant formula or a human milk for feeding an infant, wherein the infant has blood group A, B, or AB according to the ABO blood group system. Preferably the nutritional supplement is for supplementing human milk of a mother who has blood group O according to the ABO blood group system.

The invention also concerns a method for preparing a nutritional composition comprising supplementing milk from a mother that has a) Lewis blood type Le(a+/b-) according to the Lewis blood typing system and/or b) Lewis blood type Le(x+/y-) according to the Lewis blood typing system with a composition comprising 2-fucosylated oligosaccharides. Preferably in this method, the nutritional composition is for an infant that has a) Lewis blood type Le(a-/b+) or Le(a-/b-) according to the Lewis blood typing system and/or b) Lewis blood type Le(x-/y+) or Le(x-/y-) according to the Lewis blood typing system.

In one embodiment the invention also concerns a method for preparing a nutritional composition comprising supplementing milk from a mother that has blood group O according to the ABO blood group system with a composition comprising 2-fucosylated oligosaccharides. Preferably in this method, the nutritional composition is for an infant that has blood group A, B, or AB according to the ABO blood group system.

Preferably the 2-fucosylated oligosaccharides comprise alpha-1,2-fucosyl oligosaccharides, preferably 2'-fucosyllactose and/or lacto-N-fucopentaose I. 2'-Fucosyllactose is also abbreviated as 2'-FL and can also be represented by Fuc-α-(1→2)Gal-β-(1→4)-Glc. Lacto-N-fucopentaose I is also abbreviated as LNFP I and can also be represented by Fuc-α-(1→2)Gal-β-(1→3)-GlcNAc-β-(1→3)-Gal-β-(1→4)-Glc. Both substances are commercially available for instance from Sigma-Aldrich. Alternatively, they can be isolated from human milk, for example as described in Andersson & Donald, 1981, J Chromatogr. 211:170-1744, or produced by genetically modified micro-organisms, for example as described in Albermann et al, 2001, Carbohydrate Res. 334:97-103.

Preferably, the nutritional composition fed to the infant comprises 100 mg to 2 g 2-fucosylated oligosaccharides per 100 ml, more preferably 150 mg to 1.8 g, even more preferably 200 mg to 1.5 g, even more preferably 400 mg to 1 g, even more preferably 500 mg to 1 g 2-fucosylated oligosaccharides per 100 ml. Based on dry weight, the composition preferably comprises 0.6 wt% to 15 wt% 2-fucosylated oligosaccharides, more preferably 1 wt% to 12 wt%, even more preferably 1.3 wt% to 10 wt%, even more preferably 2.5 wt% to 6.5 wt%, even more preferably 3.3 wt% to 6.5 wt%. A lower amount of 2-fucosylated oligosaccharides will be less effective in preventing from infections by pathogens, whereas a too high amount will result in unnecessary high costs of the product.

The nutritional supplement preferably comprises at least 10 wt% 2-fucosylated oligosaccharides based on dry weight of the supplement. No other compounds are needed but could be allowed. In a nutritional supplement the 2-fucosylated oligosaccharides are preferably mixed with an inert carrier or bulking agent, for example maltodextrin. Preferably the nutritional supplement comprises 10 wt% to 90 wt% 2-fucosylated oligosaccharides, preferably 15 wt% to 80 wt%, preferably 25 wt% to 75 wt%, preferably 30 wt% to 65 wt%, preferably 40 wt% to 55 wt%. Preferably the nutritional supplement is in the form of a powder. Preferably such an amount of the powdered nutritional supplement is added to human milk as to arrive at a supplemented human milk that contains 100 mg to 2 g 2-fucosylated oligosaccharides per 100 ml, more preferably 150 mg to 1.8 g, even more preferably 200 mg to 1.5 g, even more preferably 400 mg to 1 g, even more preferably 500 mg to 1 g 2-fucosylated oligosaccharides per 100 ml. For example the nutritional supplement is packed in a sachet, preferably of a bout 2 g per sachet. Preferably 1, 2, 3 or 4 sachets are added per 100 ml breast milk.

### Non-digestible oligosaccharides other than 2-fucosylated oligosaccharides

The nutritional composition preferably comprises non-digestible oligosaccharides (NDO) other than 2-fucosylated oligosaccharides. Preferably the NDO other than 2-fucosylated oligosaccharides stimulate the growth of bifidobacteria and/or lactobacilli, more preferably bifidobacteria. An increased content of bifidobacteria and/or lactobacilli stimulate the formation of a healthy intestinal microbiota. The NDO are preferably not or only partially digested in the intestine by the action of acids or digestive enzymes present in the human upper digestive tract, in particular in the small intestine and stomach, and are mainly fermented by the human intestinal microbiota. For example, sucrose, lactose, maltose and the common maltodextrins are considered digestible.

Preferably the present composition comprises non-digestible oligosaccharides with a DP in the range of 2 to 250, more preferably 2 to 60. The non-digestible oligosaccharide is preferably at least one, more preferably at least two, preferably at least three selected from the group consisting of fructo-oligosaccharides, galacto-oligosaccharides, xylo-oligosaccharides, arabino-oligosaccharides, arabinogalacto-oligosaccharides, gluco-oligosaccharides, chito-oligosaccharides, glucomanno-oligosaccharides, galactomanno-oligosaccharides, mannan-oligosaccharides, sialic acid comprising oligosaccharides, and uronic acid oligosaccharides. The group of fructo-oligosaccharides includes inulins, the group of galacto-oligosaccharides includes transgalacto-oligosaccharides or beta-galacto-oligosaccharides, the group of gluco-oligosaccharides includes cyclodextrins, gentio- and nigero-oligosaccharides and non-digestible polydextrose, the group of galactomanno-oligosaccharides includes partially hydrolyzed guar gum, and the group of uronic acid oligosaccharides includes galacturonic acid oligosaccharides and pectin degradation products.

More preferably the present composition comprises at least one, more preferably at least two, most preferably three selected from the group consisting of fructo-oligosaccharides, beta-galacto-oligosaccharides and uronic acid oligosaccharides. More preferably the composition comprises beta-galacto-oligosaccharides.

In a preferred embodiment the composition comprises a mixture of inulin and short chain fructo-oligosaccharides. In a preferred embodiment the composition comprises a mixture of galacto-oligosaccharides and fructo-oligosaccharides selected from the group consisting of short chain fructo-oligosaccharides and inulin, more preferably inulin. A mixture of at least two different non-digestible oligosaccharides advantageously stimulates the beneficial bacteria of the intestinal microbiota to a greater extent. Preferably the weight ratio in a mixture of the two different non-digestible oligosaccharides, preferably galacto-oligosaccharides and fructo-oligosaccharide, is between 25 and 0.05, more preferably between 20 and 1. Galacto-oligosaccharides, preferably beta-galacto-oligosaccharides, are more capable of stimulating bifidobacteria. Preferably the present composition comprises galacto-oligosaccharides, preferably beta-galacto-oligosaccharides, with a degree of polymerization (DP) of 2 to 10 and/or fructo-oligosaccharides with a DP of 2 to 60.

The galacto-oligosaccharides preferably are beta-galacto-oligosaccharides. In a particularly preferred embodiment the present composition comprises beta-galacto-oligosaccharides ([galactose]n-glucose; wherein n is an integer ranging from 2 to 60, i.e. 2, 3, 4, 5, 6, ...., 59 ,60; preferably n is selected from 2, 3, 4, 5, 6, 7, 8, 9, and 10), wherein the galactose units are in majority linked together via a beta linkage. Beta-galacto-oligosaccharides are also referred to as trans-galacto-oligosaccharides (TOS). Beta-galacto-oligosaccharides are for example sold under the trademark Vivinal^{(™)} (Borculo Domo Ingredients, Netherlands). Another suitable source is Bi2Munno (Classado). Preferably the TOS comprises at least 80 % beta-1,4 and beta-1,6 linkages based on total linkages, more preferably at least 90 %.

Fructo-oligosaccharide is a NDO comprising a chain of beta-linked fructose units with a DP or average DP of 2 to 250, more preferably 2 to 100, even more preferably 10 to 60. Fructo-oligosaccharide includes inulin, levan and/or a mixed type of polyfructan. An especially preferred fructo-oligosaccharide is inulin. Fructo-oligosaccharide suitable for use in the compositions is also commercially available, e.g. Raftiline®HP (Orafti). Preferably the fructo-oligosaccharide has an average DP above 20.

Uronic acid oligosaccharides are preferably obtained from pectin degradation products. Hence the present composition preferably comprises a pectin degradation product with a DP of 2 to100. Preferably the pectin degradation product is prepared from apple pectin, beet pectin and/or citrus pectin. Preferably the uronic acid oligosaccharide is a galacturonic acid oligosaccharide. Preferably the composition comprises FL and one of the group selected from galacto-oligosaccharide and uronic acid oligosaccharide.

Besides 2-fucosylated oligosaccharides, most preferably the composition comprises beta-galacto-oligosaccharide, fructo-oligosaccharide and a uronic acid oligosaccharide. It was found that such a combination acts synergistically with fucosyllactose, in particular 2'-fucosyllactose. The weight ratio beta-galacto-oligosaccharide : fructo-oligosaccharide : uronic acid oligosaccharide is preferably (20 to 2) : 1 : (1 to 20), more preferably (20 to 2) : 1 : (1 to 10), even more preferably (20 to 2) : 1 : (1 to 3), even more preferably (12 to 7) : 1 : (1 to 2). Most preferably the weight ratio is about 9 : 1 : 1.1..

Preferably, the nutritional composition comprises 200 mg to 4 g non-digestible oligosaccharides, including 2-fucosylated oligosaccharides, per 100 ml, more preferably 400 mg to 3.5 g, even more preferably 500 mg to 3 g, even more preferably 800 mg to 2.5 g even more preferably 1 g to 2 g non-digestible oligosaccharides per 100 ml. Based on dry weight, the composition preferably comprises 1 wt% to 25 wt% non-digestible oligosaccharides including 2-fucosylated oligosaccharides, more preferably 2.5 wt% to 20 wt%, even more preferably 3.3 wt% to 18 wt%, even more preferably 5 wt% to 15 wt%, even more preferably 6.5 wt% to 13 wt%. A lower amount of non-digestible oligosaccharides will be less effective in stimulating the beneficial bacteria in the microbiota, whereas a too high amount will result in side-effects of bloating and abdominal discomfort.

### Nutritional composition

The nutritional composition of the present invention is not human milk. The present nutritional composition is preferably enterally administered, more preferably orally.

The present nutritional composition is preferably an infant formula or a toddler formula, preferably to be administered after birth up to 6 years more preferably after birth until 36 months of age. The present nutritional composition can be advantageously applied as a complete nutrition for infants. The present composition preferably comprises a lipid component, protein component and carbohydrate component and is preferably administered in liquid form. The present invention includes dry food, preferably a powder, which is accompanied with instructions as to admix said dry food mixture with a suitable liquid, preferably with water.

The present nutritional composition preferably comprises lipid, protein and digestible carbohydrate, wherein the lipid component provides 5 to 50% of the total calories, the protein component provides 5 to 50% of the total calories, and the digestible carbohydrate component provides 15 to 85% of the total calories. Advantageously, the lipid component provides 20 to 50% of the total calories, the protein component provides 5 to 30% of the total calories, and the digestible carbohydrate component provides 30 to 70% of the total calories. Preferably, the lipid component provides 35 to 50% of the total calories, the protein component provides 7.5 to 12.5% of the total calories, and the digestible carbohydrate component provides 40 to 55% of the total calories. For calculation of the % of total calories for the protein component, the total of energy provided by the proteins, peptides and amino acids needs to be taken into account.

The nutritional composition preferably comprises at least one lipid selected from the group consisting of animal lipid, excluding human lipids, and vegetable lipids. Preferably the present composition comprises a combination of vegetable lipids and at least one oil selected from the group consisting of fish oil, animal oil, algae oil, fungal oil, and bacterial oil. The present composition preferably comprises long chain polyunsaturated fatty acids (LC-PUFA). LC-PUFA are fatty acids or fatty acyl chains with a length of 20 to 24 carbon atoms, preferably 20 or 22 carbon atoms comprising two or more unsaturated bonds. More preferably the present composition comprises eicosapentaenoic acid (EPA, n-3), docosahexaenoic acid (DHA, n-3) and/or arachidonic acid (ARA, n-6).

Preferably the present composition comprises at least 0.1 wt.%, preferably at least 0.25 wt.%, more preferably at least 0.6 wt.%, even more preferably at least 0.75 wt.% LC-PUFA with 20 and 22 carbon atoms based on total lipid content.

The content of LC-PUFA, particularly the LC-PUFA with 20 and 22 carbon atoms, preferably does not exceed 6 wt%, more preferably does not exceed 3 wt.% of the total lipid content as it is desirable to mimic human milk as closely as possible. The LC-PUFA may be provided as free fatty acids, in triglyceride form, in diglyceride form, in monoglyceride form, in phospholipid form, or as a mixture of one of more of the above. The present composition preferably comprises between 5 and 75 wt.% polyunsaturated fatty acids based on total fat, preferably between 10 and 50 wt.%.

The protein in the nutritional composition is preferably selected from the group consisting of non-human animal proteins (preferably milk proteins), vegetable proteins (preferably soy protein and/or rice protein), hydrolysates thereof, free amino acids and mixtures thereof. The nutritional composition preferably contains casein, whey, hydrolyzed casein and/or hydrolyzed whey protein. Preferably the protein comprises intact proteins, more preferably intact bovine whey proteins and/or intact bovine casein proteins.

The nutritional composition preferably contains digestible carbohydrates selected from the group consisting of sucrose, lactose, glucose, fructose, corn syrup solids, starch and maltodextrins, more preferably lactose.

In view of the above, it is also important that the liquid food does not have an excessive caloric density, however still provides sufficient calories to feed the infant. Hence, the liquid food preferably has a caloric density between 0.1 and 2.5 kcal/ml, even more preferably a caloric density of between 0.5 and 1.5 kcal/ml, most preferably between 0.6 and 0.8 kcal/ml.

Preferably the nutritional composition comprises nucleotides and/or nucleosides, more preferably nucleotides. Preferably, the composition comprises cytidine 5'-monophospate, uridine 5'-monophospate, adenosine 5'-monophospate, guanosine 5'-monophospate, and/or inosine 5'-monophospate, more preferably cytidine 5'-monophospate, uridine 5'-monophospate, adenosine 5'-monophospate, guanosine 5'-monophospate, and inosine 5'-monophospate. Preferably the composition comprises 5 to 100, more preferably 5 to 50 mg, most preferably 10 to 50 mg nucleotides and/or nucleosides per 100 gram dry weight of the composition.

### Infant

The present method is advantageously applied to a human infant of 0-36 months, more preferably to a human infant of 0-18 months, more preferably to a human infant of 0-12 months, even more preferably to a human infant of 0-6 months. An infant of 0-36 months includes a toddler. In one embodiment a toddler has the age of more than 12 months to 36 months or more than 18 months to 36 months. To decrease the risk of infection by pathogens to a minimum it is advantageous to supplement the nutrition with 2-fucosylated oligosaccharides as early as possible.

Preferably the blood type of the infant is determined on the basis of the Lewis a and Lewis b antigens. In case the blood type of the infant is determined on the basis of the Lewis x and Lewis y antigens, preferably the infant has Lewis blood type Le(x-/y+).

### FIGURES

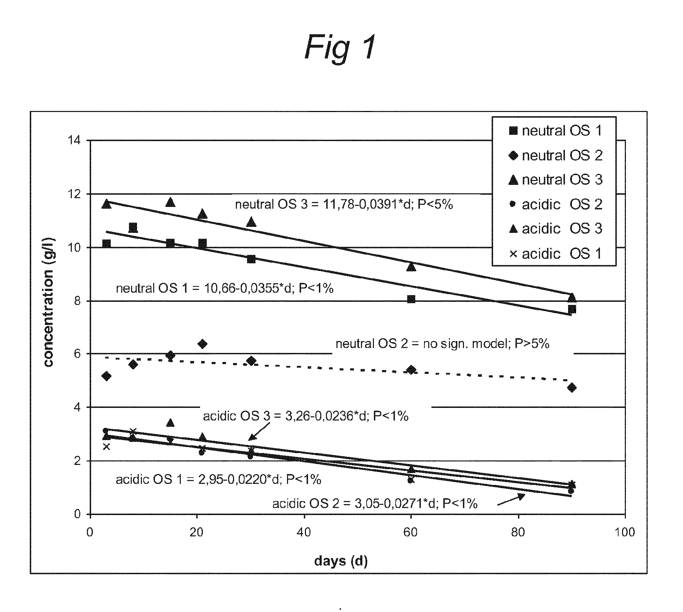

Fig. 1 shows the variation of neutral and acidic oligosaccharides within milk groups 1-3. All data points represent mean values of the corresponding oligosaccharide concentrations at seven times postpartum. However, statistical calculations are based on individual values. Straight lines indicate significant trends (P<5%); the dotted trend line indicates not significant variations during time.

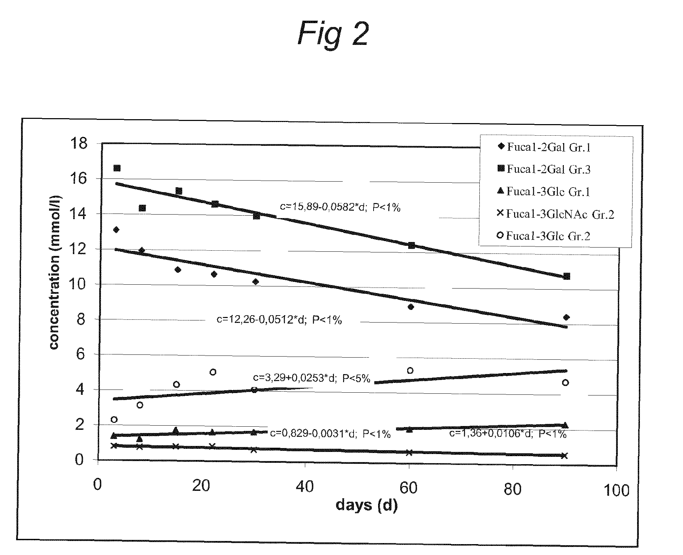

Fig. 2 shows the variation of α1,2-, α1,4- and α1,3-linked fructose moieties. All data points represent mean values of the corresponding oligosaccharide concentrations at seven times postpartum. However, statistical calculations are based on individual values. Fucα1-2Gal: α1,2-linked fucosyl moieties as detected in milk groups 1 or 3; Fucα1-4GlcNAc: α1,4-linked fucosyl moieties as detected in milk groups 1 or 2; Fucα1-3Glc: α1,3-fucosyl moieties linked to subterminal glucose as detected in milk groups 1, 2, 3; Fucα1-3GlcNAc: α1,3-fucosyl moieties linked to GlcNAc as detected in milk groups 1, 2, 3; No significant trends (P>5%) were found with Fucα1-4GlcNAc: milk groups 1 and 2, Fucα1-3Glc: milk group 3, Fucα1-3GlcNAc: milk groups 1 and 3.

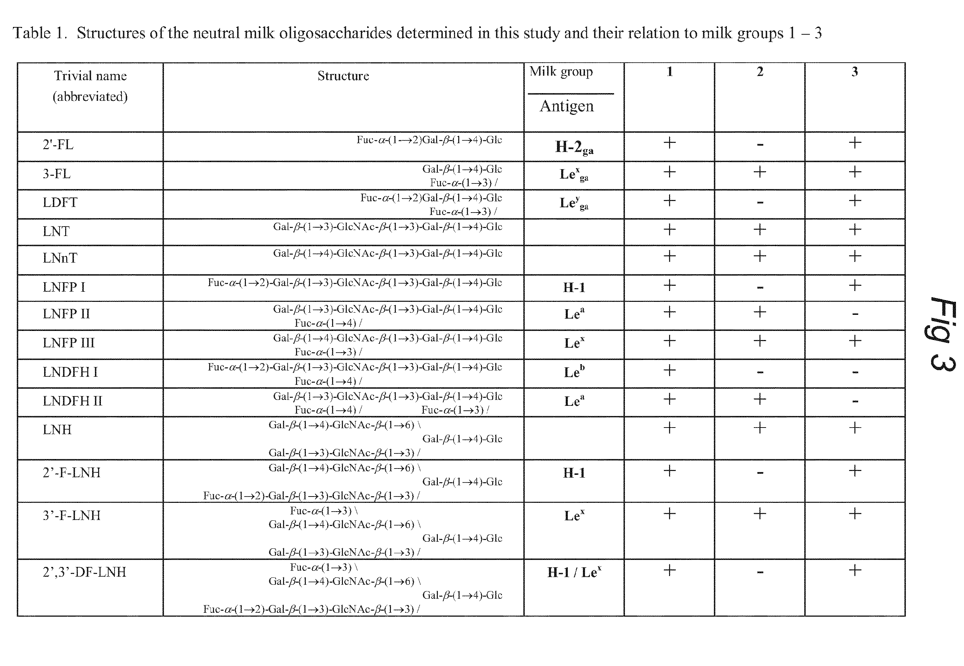

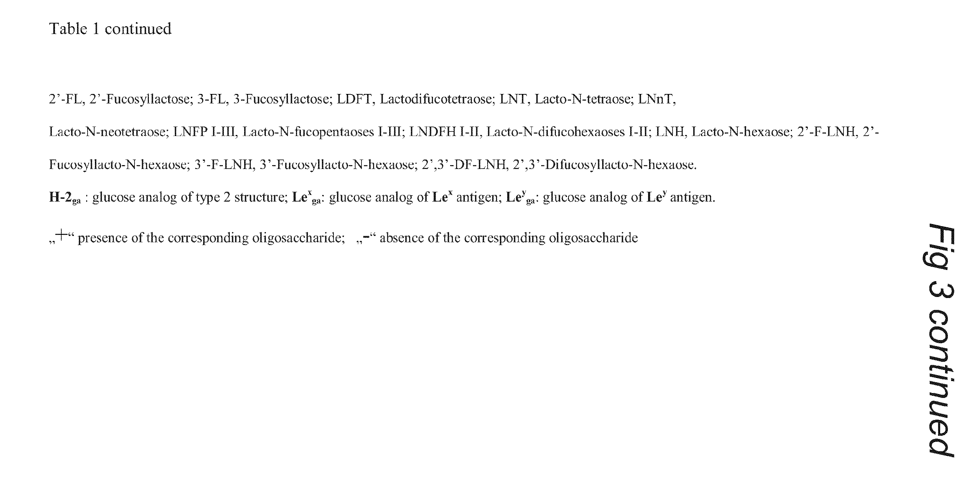

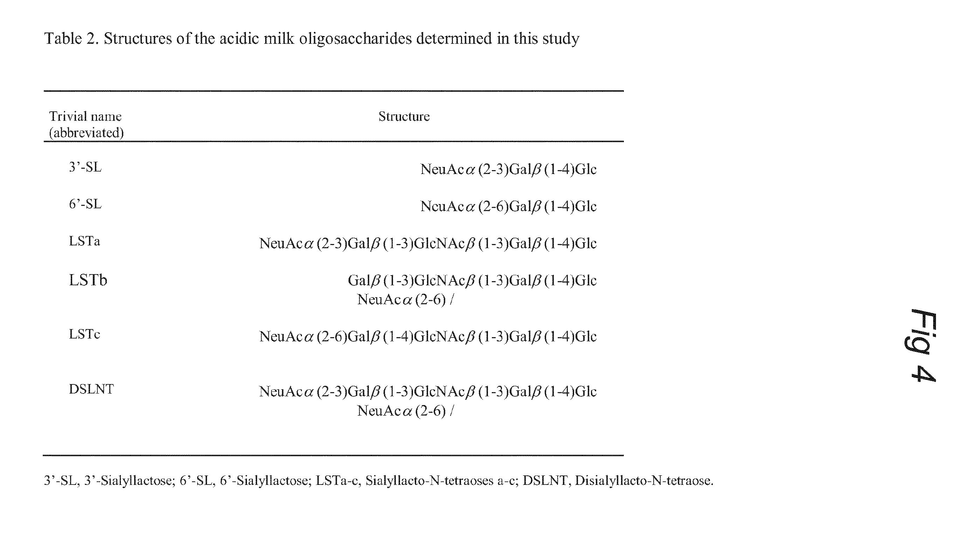

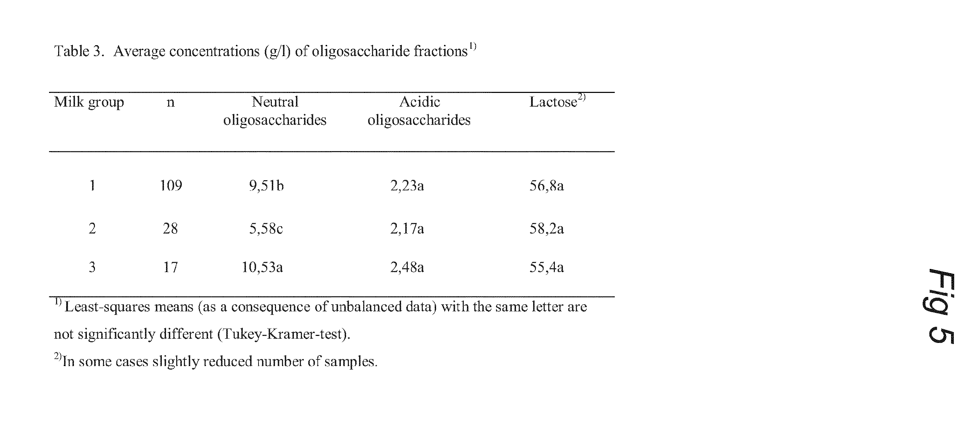

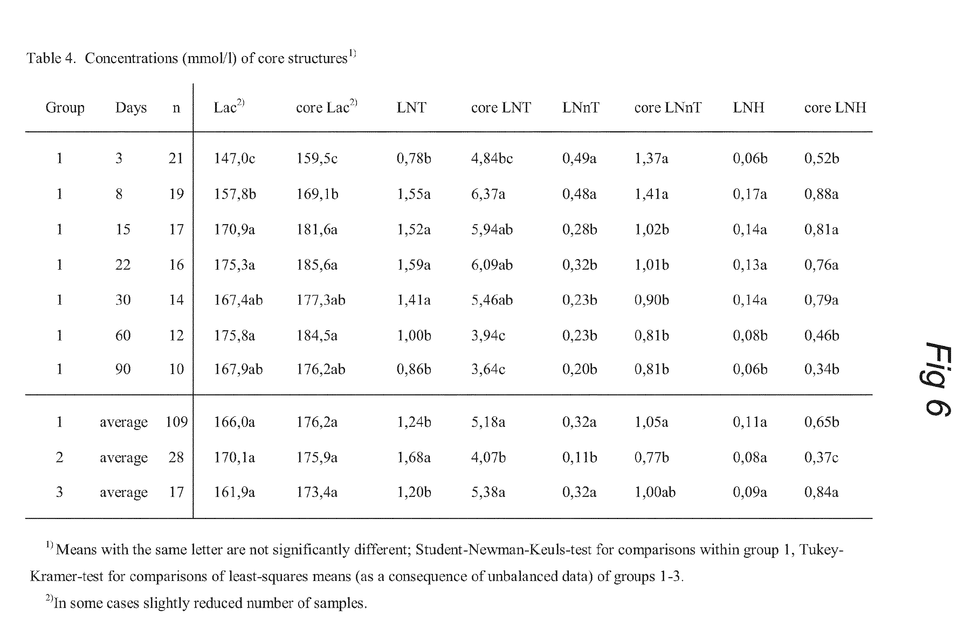

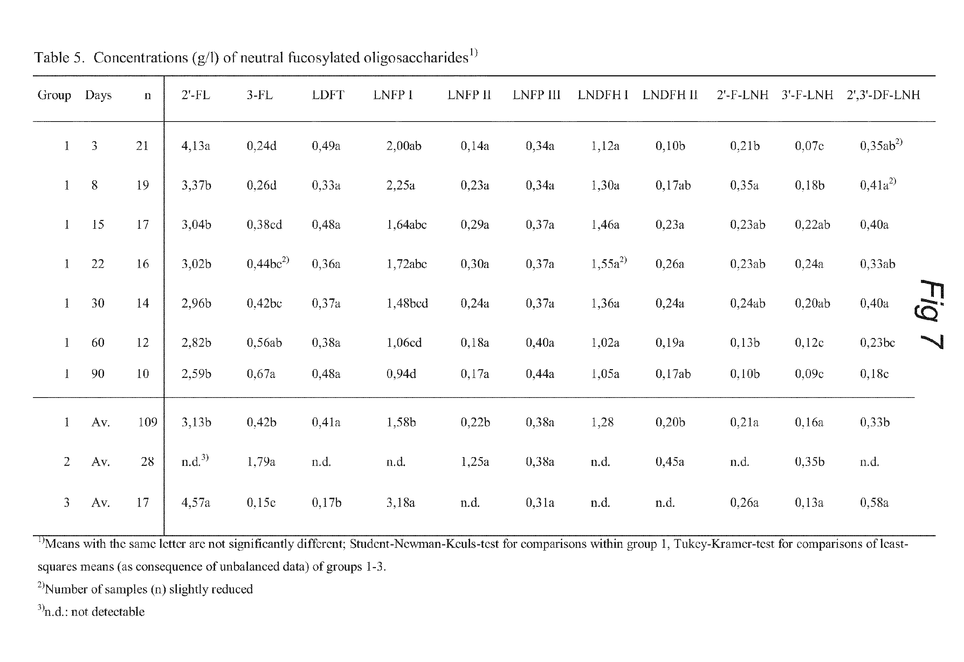

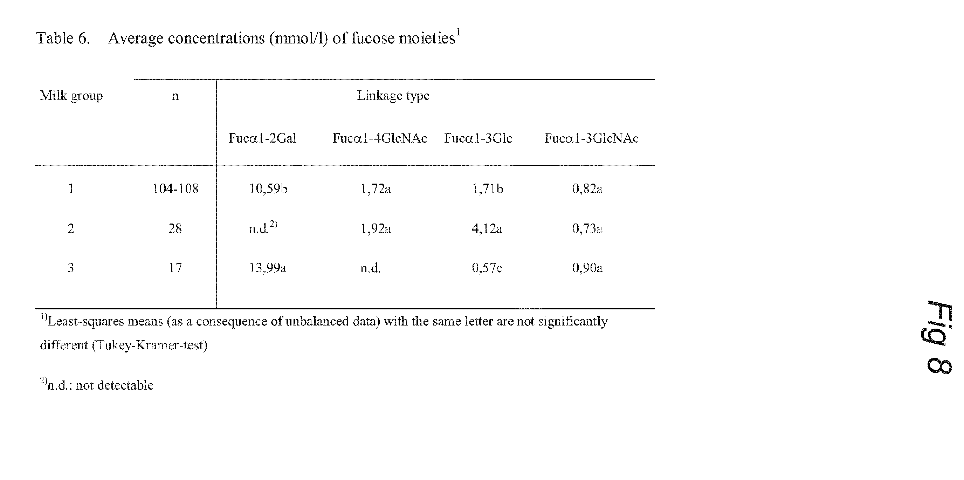

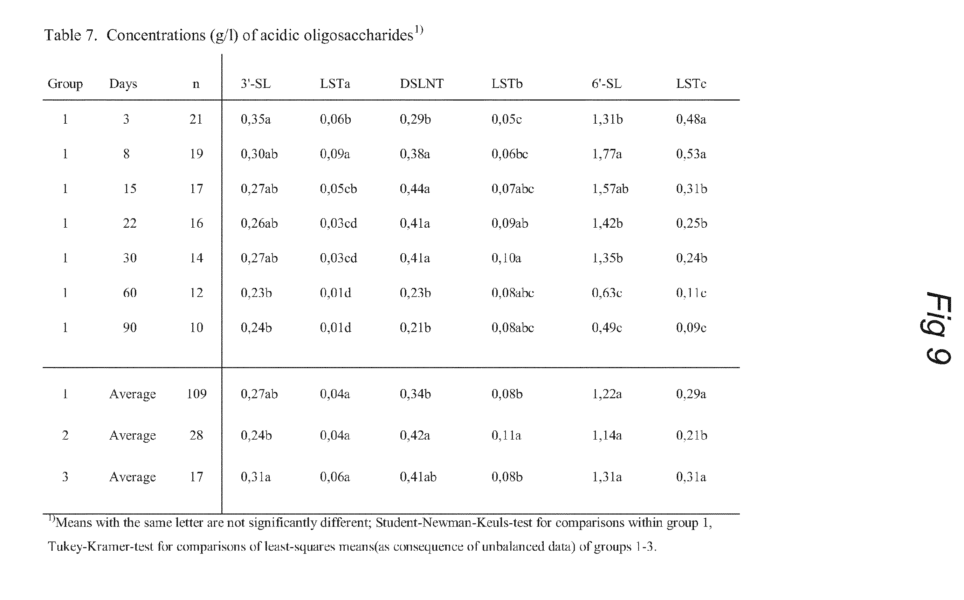

Figures 3-9 show tables 1-7 referred to in the examples.

### EXAMPLES

### Example 1

### Materials and Methods

### Serologic tests

Lewis blood groups of the women were determined within 3 days post partum on the day of blood sampling by a haemagglutination tube test. Haemagglutination was examined using corresponding erythrocyte suspensions (3%-5% erythrocytes suspended in 0,9% NaCl ) and monoclonal anti-Le^{a} and anti-Le^{b} antibodies (Immucor, Rödermark, Germany and BAG, Lich, Germany). Incubation was performed at room temperature for 15 min. Due to discrepancies between serologic tests and chromatographic profiles, some heamagglutination tests were repeated 18 - 25 months post partum. The women were not pregnant at that time.

### Collection of samples

All mothers had given written consent for participating in this study the design of which had been approved by the ethical committee of the university hospital of Dresden, Germany. The 30 caucasian women were living in the region of Dresden, they were between 20 and 35 years old and had given birth to healthy infants who were exclusively breast-fed during the study period. As a whole 175 breast milk samples were taken predominantly at following time intervals including seven major days: day 3, 2 - 5 days postpartum; day 8, d6 - d9; day 15, d13 - d18; day 22, d20 - d26; day 30, d28 - d33; day 60, d57 - d65; day 90, d88 - d96. In cases when mothers collected more than one milk sample corresponding to the above mentioned time intervals, all samples were analysed with oligosaccharide concentrations expressed as arithmetic mean values. In a preexamination with six Caucasian women (Lewis blood type Le(a-b+)) from the region of Frankfurt/Main, Germany, we could not find any significant oligosaccharide variation during two 24 hour periods at days 7 and 60 post partum (data not shown). Nevertheless, in order to exclude even small diurnal effects, the sampling time in this study was fixed to the morning feed. The milk samples generally were collected during the morning hours at 6-10 am by applying the mid-feed sampling technique, previously shown to be an adequate sampling technique for carbohydrate analysis. About 5-10 ml aliquots were expressed manually in the middle of feeding into plastic containers. Milk samples were immediately frozen and stored at -20°C until analysis.

### Chromatographic analysis of oligosaccharides

Sample preparation including gel permeation chromatographic purification, as well as HPAEC-analyses were performed as already described. Briefly, human milk samples were heated for 30 min at 70 °C. One millilitre of human milk was added with 0,1 ml of an aqueous solution containing the internal standards stachyose and galacturonic acid. The samples were subsequently centrifuged and ultrafiltrated (Millipore Centrifree, 30 kDa cutoff). The protein and lipid reduced samples were fractionated into lactose, neutral oligosaccharides and acidic oligosaccharides by gel permeation chromatography using Toyopearl HW 40 (S ) columns (1,6 x 80 cm TosoHaas, Stuttgart, Germany). The carbohydrate fractions were eluted with water (flow rate of 1 ml/min) and monitored by refractive index detection. The lactose fraction was discarded; the neutral and acidic fractions were analysed by HPAEC-PED. The eluents used for analysis of neutral oligosaccharides were 0-20 min, 30 mM NaOH; 20-34 min, 30-100 mM NaOH; 34-48 min, 100 mM NaOH / 0-28 mM NaOAc; 48-55 min, 100 mM NaOH / 28-200 mM NaOAc; 55-60 min, 100 mM NaOH / 200 mM NaOAc. The elution conditions for acidic oligosaccharides were 0-8 min, 100 mM NaOH / 20 mM NaOAc; 8-30 min, 100 mM NaOH / 20-80 mM NaOAc; 30-55 min, 100 mM NaOH / 80-200 mM NaOAc; 55-60 min, 100 mM NaOH / 200 mM NaOAc.

In addition, most neutral oligosaccharide fractions were analysed a second time with a different gradient (0-12 min, 60 mM NaOH; 12-16 min, 60-100 mM NaOH; 16-30 min, 100 mM NaOH / 0-28 mM NaOAc; 30-35 min, 100 mM NaOH / 28-200 mM NaOAc; 35-40 min, 100 mM NaOH / 200 mM NaOAc) allowing separation and quantification of the coeluting neutral oligosaccharides 3'FL and LNDFH I. In contrast to the above mentioned publication this additional step was necessary due to altered performances of the CarboPac PA-100 columns (Dionex, Idstein, Gemany) used during the study.

In order to monitor possible artificial hydrolytic degradation of especially susceptible sialyloligosaccharides, free N-acetyl neuraminic acid (Neu5Ac) was quantified along with acidic oligosaccharides. Neu5Ac concentrations were relatively constant (average concentration of 0,019 g/L in milk group one; data not shown). The amounts of free NeuAc were of the same magnitude as already reported and correspond to approximately 2% and 4% of oligosaccharide bound NeuAc at the beginning of lactation and after three months, respectively. Therefore significant degradation of acidic oligosaccharides due to the action of sialidases or to heat treatment can be excluded.

### Determination of lactose

250 microliters of human milk were mixed with deionized water and with 1 mL Carrez-I-solution (85 mmol/L of potassium hexacyanoferrate(II)) and 1 mL Carrez-II-solution (250 mmol/L of zinc sulfate) in a 25 mL volumetric flask. After filtration lactose concentrations of the diluted (factor 100) and protein and lipid reduced milk samples were determined using a lactose/D-galactose test combination from R-Biopharm, Darmstadt, Germany. β-galactosidase released galactose which subsequently was oxidised by galactose dehydrogenase. Lactose concentrations could be calculated with the ultraviolet absorbances of the reduced cofactor nicotinamide-adenine dinucleotide at 340 nm.

### Statistical analyses

Data that were analyzed by statistical methods either consisted of concentrations of individual oligosaccharides or of sums of various carbohydrates. Apart from total neutral and total acidic oligosaccharides (Tables 1 and 2), following total core structures and fucosylated carbohydrates were summed up, respectively: core Lac = Lac + 3-FL + 2'-FL + LDFT + 3'-SL + 6'-SL; core LNT = LNT + LNFP I + LNFP II+ LNDFH I + LNDFH II + LSTa + LSTb + DSLNT; core LNnT = LNnT + LNFP III + LSTc; core LNH = LNH + 2'-F-LNH + 3'-F-LNH + 2',3'-DF-LNH; Fucα1-2Gal = 2'-FL + LDFT + LNFP I + LNDFH I + 2'-F-LNH + 2',3'-DF-LNH; Fucα1-4GlcNAc = LNFP II + LNDFH I + LNDFH II; Fucα1-3Glc = 3-FL + LDFT + LNDFH II; Fucα1-3GlcNAc = LNFP III + 3'-F-LNH + 2',3'-DF-LNH.

The data set is two-factorially organized, in three milk groups and seven lactation times, respectively. Besides, the data set is very unbalanced due to different sample numbers. In milk group 1 the 109 samples in total are allocated to the times in a range from 10 to 21 samples, whereas in group 2 (28 samples) a lactation time is represented by 3 - 5 samples and in group 3 (17 samples) by 2 - 3 samples. Therefore several methods for analyzing the means of oligosaccharide concentrations were applied. In the case of milk group 1, an one-factorial analysis of variance (ANOVA) followed by a Student-Newman-Keuls-test to compare the mean values of lactation times was used. Comparing the averages of the three milk groups a two-factorial ANOVA with Type III sum of squares was applied and then least-squares means were calculated. So, the group averages are unbiased and completely comparable together. Differences between averages were tested with the Tukey-Kramer-method on a significance level of 5%. In both variance models the differences between the sample concentrations of the participating women per group and time yield the experimental error. Generally, oligosaccharide concentrations of milks from women of the same milk group at a given lactation period were highly variable. Due to these large interindividual variations, in same cases no significant differences between means (P>5%) were found despite seemingly clear differences.

Trends of oligosaccharide concentrations during lactation were modelled by regression analysis. Simple linear regression as well as polynomial regression of 2^{nd} and 3^{rd} degree were fitted and tested on significant coefficients of regression. The model was accepted if the coefficients of regression were significant (P<5%). All analyses of regression were carried out with the individual values, although in the figures due to a better clarity only the means of lactation times are drawn. All computations were done using the SAS System (SAS Institute Inc. 2002-2003. SAS/STAT. Release 9.1. SAS Institute Inc., Cary, NC, USA).

### Results

### Lewis blood groups and oligosaccharides profiles

According to heamagglutination tests twenty-two donors (73%) revealed Lewis blood type Le(a-b+), five donors (17%) were determined as Lewis blood type Le(a+b-), whereas three women (10%) were Le(a-b-) donors. These proportions were in a similar range as the frequencies of Lewis blood groups among European populations.

Applying a HPAEC method fourteen neutral oligosaccharides listed in Table 1 could be determined. In accordance with their Lewis blood group Le(a-b+), milk samples of twenty-two donors exhibited all these fourteen structures including α1,2-, α1,4-, and α1,3-fucosyloligosaccharides. Milk samples revealing this oligosaccharide pattern had been assigned to milk group one. Milk samples of the five nonsecretor women (Lewis blood type Le(a+b-)) lacked 2'-FL, LDFT, LNFP I, LNDFH I, 2'-F-LNH and 2',3'-DF-LNH and could be assigned to the second milk group, because no α1,2-fucosyloligosaccharides were detected (Table 1). In the milk samples of three mothers with Lewis blood type Le(a-b-) α1,2- and α1,3-fucosyloligosaccharides were found, whereas LNFP II, LNDFH I and LNDFH II with α1,4 fucose residues were missing. One of these women (B.A.) also lacked LDFT, a fucosylated component typical for this milk group, and 3-F-LNH, which was normally detected in all milk samples. In spite of these minor deviations all three mothers were assigned to milk group three (Table 1). None of the thirty women in this study exhibited an oligosaccharide profile without α1,2-linked and α1,4-linked fucosyloligosaccharides, corresponding to milk group four. This is in agreement with the low prevalence (1%) of the genotype se/se and le/le among Caucasian women.

### Carbohydrate fractions

In Table 3 average concentrations of the major carbohydrate fractions are shown. Lactose concentrations determined enzymatically were similar in milk samples of all three milk groups. During the study period concentrations remained constant in mature milk (milk group one: 57 - 60 g/L; data not shown), whereas colostral milk at days 3 postpartum contained significantly lower amounts of lactose (average concentration of 50,3 g/L in milk group one).

The sum of the fourteen major neutral oligosaccharides determined by chromatographic analysis approximately represents the neutral oligosaccharide fraction of human milk (Table 1). This carbohydrate fraction is relatively abundant in secretor milks, especially in milk group three, whereas nonsecretors only produce approximately the half amount. In Figure 1 variation of neutral oligosaccharides during the course of lactation are shown. Concentrations of neutral oligosaccharides in secretor milks steadily decrease during the first 90 days postpartum as indicated by two significant declining straight lines calculated by regression analysis. In contrast, neutral oligosaccharides in nonsecretor milks seem to remain relatively constant during the study period. In contrast to nearly constant levels of neutral sugars in nonsecretor milks, secretors steadily produce lower amounts of these sugars during the first three months postpartum.

In addition to neutral oligosaccharides, six major nonfucosylated acidic oligosaccharides, 3'-SL, 6'-SL, LSTa, LSTb, LSTc, DSLNT, as shown in Table 2, could be determined chromatographically. The sum of these carbohydrates approximately represents the acidic oligosaccharide fraction of human milk. Because of the importance of the Lewis blood group system these sugar also were examined separately according to the three milk groups. Milk samples of all three milk types exhibited the above mentioned six acidic oligosaccharides. The quantities of the acidic oligosaccharide fractions did not differ significantly among the three milk groups (Table 3). In addition, all milk groups exhibited a similar roughly threefold decrease of the acidic sugar concentrations during the study period.

### Core structures

The molar concentrations of the core structures detected in samples of all three milk groups, lactose, LNT, LNnT and LNH, are shown in Table 4. Total core structures, including unmodified, fucosylated or sialylated oligosaccharides, are compared to the corresponding not decorated core structures. Lactose, that can be interpreted as glucose analogue of type 2 chains, was by far the most abundant core structure exceeding LNT, a type 1 structure, on average by a factor of 34 (Table 4). LNnT, a typical type 2 structure, and LNH were detected in even lower quantities. Lactose was found to be the prevalent form (about 95%) of the core structure lactose. In contrast, unmodified LNT, LNnT and LNH varied between 17% and 31 % in relation to the corresponding total core structures. Obviously, fucosylated and sialylated sugars are the predominant forms of these three basic components.

LNT found in nonsecretor milks was expressed in higher amounts than in secretor samples, but when comparing total core LNT it turned out that nonsecretor mothers synthesized significantly lower amounts of this core structure (Table 4). Nonsecretor women also produced significantly lower concentrations of the core structures LNnT and LNH, roughly 80 to 60% of the amounts detected in secretor milks. In contrast, core lactose did not differ significantly among the three milk groups. Concentrations of core LNT, core LNnT and core LNH in milk group one significantly decreased during the study period. However, colostrum exhibited significantly lower amounts of core LNT and core LNH when compared to transitional milk at day 8 postpartum. For reasons of clarity time effects are shown in Table 4 only within milk group one. Milk groups two and three revealed similar trends during the course of lactation. Due to low sample numbers these changes not always were juged as significant (data not shown).

### Fucosylated neutral oligosaccharides

Average concentrations of eleven fucosylated neutral oligosaccharides that were quantified in this study are shown in Table 5. The major fucosylated carbohydrates in samples of milk groups one and three, that is secretor milks, are 2'-FL, LNFP I and, in the case of milk group one, LNDFH I. Nonsecretors predominantly biosynthesised 3-FL and LNFP II. These carbohydrates as well as LNDFH II and 3'-F-LNH were significantly higher expressed than in secretor milks. During the study period concentrations of sugars typical for secretor milks, 2'-FL, LNFP I, 2'-F-LNH and 2',3'-DFLNH, roughly decreased twofold, with the exception of LDFT, which did not change significantly, and LNDFH I. 3-FL was the only oligosaccharide steadily increasing in all milk groups about twofold during the first three months of lactation. LNFP III also seemed to increase in secretor milks although this variation was not statistically significant. The concentrations of LNFP II, LNDFH I, LNDFH II and 3'-F-LNH in milk group one samples seem to increase to maximum levels during the first month postpartum. Mature milks after 3 months contained similar lower amounts of these sugars as colostral milks.

Table 6 and Figure 2 summarize the variation of fucosylated oligosaccharides during the course of lactation with regard to the three milk groups. Oligosaccharides were summed on a molar basis according to four different linkage types: α1,2-fucose linked to terminal galactose (Fucα1-2Gal), α1,4-fucose linked to subterminal N-acetylglucosamine (Fucα1-4GlcNAc), α1,3-fucose linked to reducing glucose (Fucα1-3Glc), α1,3-fucose linked to subterminal GlcNAc (Fucα1-3GlcNAc). The former α1,2-fucose moieties, typical for secretor milks were expressed in milk group three in significantly higher concentration than in milk group one. In both milk groups these concentrations declined during the study period as indicated by significant straight lines of regression in Figure 2. Fucose moieties with α1,4-bonds, products of the Lewis gene, were equally expressed in groups one and two (Table 6). At two and three weeks after birth their concentrations seem to attain maximum levels with colostral concentrations resembling those of mature milk after two and three months postpartum. However, these trends were not significant and therefore are not shown in Figure 2. Fucose moieties that are α1,3-linked to reducing glucose were detected in all three milk groups, especially in milk samples of nonsecretors representing the prevalent form of fucose. Increasing amounts of this fucose moiety were found during the study period, being significant in the case of milk groups one and two (Figure 2). In all milk groups equally low levels of α1,3-fucose residues linked to GlcNAc were detected, that remained relatively constant during the first 90 days of lactation.

### Acidic sialylated oligosaccharides

Mean concentrations of the six acidic oligosaccharides determined in this study are shown in Table 7. 6'-SL quantitatively was by far the most important acidic carbohydrate in all milks. Medium amounts of 3'-SL, LSTc and DSLNT were detected, whereas LSTa and LSTb did not occur at concentration levels above 0,1 g/L. Average concentrations of individual acidic sugars, though statistically different in some cases, did not vary to a great extent among the three milk groups, thus confirming the results of the whole carbohydrate fraction (Table 3).

Analogously, time effects, as shown in Table 7 with milk group one, were similar to tendencies in the other milk groups (data not shown and Figure 1). Concentrations of 6'-SL peaked in transitional milk at day 8, and declined at least threefold until 90 days after birth. LSTc, also containing a Neu5Acα2-6Gal-linkage, decreased in a similar manner approximately fivefold during the study period. 3'-SL was expressed in mature milk at a relatively constant level after a significant decrease in the first phase of lactation. Concentrations of LSTa, a minor acidic sugar bearing a Neu5Acα2-3Ga1-linkage, decreased after one week postpartum, so that it could not be detected in several milk samples after one month. In contrast, concentrations of LSTb exhibiting a Neu5Acα2-6GlcNAc-linkage increased during the first month and remained relatively constant thereafter. DSLNT, the only disialylated carbohydrate analyzed and a kind of hybrid structure between LSTa and LSTb, exhibited a maximum time curve.

### Discussion

Apart from lactose, 6 acidic sialylated oligosaccharides, 3 neutral core structures and 11 neutral fucosylated carbohydrates could be determined during the course of lactation applying high-pH anion-exchange chromatography. In this study we found differences of individual oligosaccharides with regard to the three main Lewis blood group dependant milk groups and with lactational periods. Up to now, only few comparisons of several oligosaccharides among two milk groups have been performed.

### Fucosyloligosaccharides

The predominant individual oligosaccharides in milk samples of secretor mothers are 2'-FL and LNFP I, consistent with qualitative and quantitative results of earlier reports. The highest concentrations of α1,2-linked fucosyloligosaccharides, especially of 2'-FL, LNFP I and 2,3-DF-LNH (Table 5 and Figure 2), were found in samples of milk group three (Le(a-b-). It is generally acknowledged that these carbohydrates are synthesized by the secretor enzyme (FucT II) accepting preferentially type 1 structures. LNT, a type 1 structure, represents the precursor of LNFP I as well as lactose, that can be defined as the glucose analog to type 2 chains (Galβ1-4GlcNAc), is the precursor of 2'-FL. Although molar concentrations of the acceptor core lactose prevail the acceptor core LNT with a factor of approximately 34 in milk group one, the amount of 2'-FL and LDFT, the secretor enzyme products from lactose, surpass the amount of LNFP I and LNDFH I, the corresponding products from LNT, only 2,3 times (data not shown); hence an at least tenfold preference for type 1 chains can be deduced. In addition, we found that lacto-N-hexaose was fucosylated to the same extent as LNT, which means that it was equally accepted by the Lewis enzyme (data not shown). Ordinary type 2 chains obviously are not at all α1,2-fucosylated at the terminal galactose in milk sugars, because free carbohydrates containing H-2 antigens, for example 2'-fucosyllacto-N-neotetraose, had not been detected so far. In agreement with previous reports, the concentration of the sum of α1,2-fucosyloligosaccharides decreased significantly within the first three months of lactation (Figure 2). Hence, one could conclude that the activity of the secretor enzyme is generally reduced during the course of lactation. However, since the acceptor core structures LNT and LNH with the exception of lactose, declined in a similar manner during the study period (Table 4), it remains doubtful whether FucT II really became less active on type 1 structures.

The sum of α1,4-fucosyloligosaccharides detected (LNFP II, LNDFH I and LNDFH II) seemed to be slightly higher, although not significant, in samples of nonsecretors (milk group two) compared to milk group one (Table 6). These carbohydrates are the main product of the Lewis enzyme (FucT III), which preferentially accepts type 1 chains resulting in Le^{a} or Le^{b} antigens. We argue that nonsecretor women reveal a higher activity of the Lewis enzyme in comparison with secretors, because although the precursor core LNT being present in significant lower amounts (about 80%, Table 3), the Lewis products tended to even higher concentrations. The Lewis enzyme was found to be about 100 times more efficient on H-type 1 compared to H-type 2 substrates. Therefore we conclude that Le^{x} antigens, α1,3-fucose moieties linked to subterminal GlcNAc from type 2 chains, as found in LNFP III, 3'-F-LNH and 2',3'-DF-LNH, are not or only to a minor extent products of the Lewis enzyme (FucT III). The finding of similar concentrations of these α1,3-fucosyloligosaccharides in samples of Le(a-b-) women (Table 6), which do not express FucT III, strongly supports this argument. It can be assumed that these sugars are biosynthesized by a third fucosyltransferase, for example the so-called plasma-enzyme (FucT VI) that had been detected in human milk (Mollicone et al. 1990). Even further fucosyltransferases could be involved, since human FucTs IV, V, VII, VIII, IX are also known to act as α1,3-fucosyltransferases.

3-FL, LDFT and LNDFH II, exhibiting fucose moieties α1,3-linked to reducing glucose, were found in samples of milk group one in approximately threefold higher amounts in comparison to milk group three (Tables 5 and 6). Obviously, women with no active Lewis enzyme produce only minor amounts of these carbohydrates. The Lewis enzyme isolated from human milk as well as a soluble form of the recombinant FucT III, have been reported to link fucose also to the 3-O position of the reducing glucose of human milk oligosaccharides in reasonable amounts. Therefore we assume that the major portion of these α1,3-fucosyloligosaccharides are the product of the Lewis enzyme, too. A smaller portion should be due to further fucosyltransferases. The especially high amounts of the Fucα1-3Glc motif in milk group two, that are caused by high quantities of 3-FL and LNDFH II, can only be partially explained with an increased activity of the Lewis enzyme. Assuming a common enzyme involved in the biosynthesis of Fucα1-4GlcNAc- and Fucα1-3Glc-motifs, similar variations during the course of lactation can be expected. However, α4-fucosyloligosaccharides seem to exhibited maximal values during the first month after birth, whereas sugars containing the Fucα1-3Glc-linkage significantly increased during the study period. It is unknown, whether the acceptor specificity of the Lewis enzyme could be shifted towards the glucose analog of type 2 chains in nonsecretor mothers during the course of lactation.

### Acidic oligosaccharides

In contrast to neutral sugars, the six acidic oligosaccharides determined in this study did not exhibit important differences among the three milk groups. This is not unexpected since these sugars lacked fucose moieties. Several α1,2- and α1,4-fucosylated acidic oligosaccharides that had been detected in human milk should considerably vary among the various milk groups, but such components were not quantified in this study. Concentrations of 6'-SL, the predominant acidic sugar in our study, exceeded the amounts reported from other groups at least twofold, whereas the concentrations of DSLNT were relatively low. Although quantitative results from different studies including our report revealed large differences, a significant decrease of the acidic sugar fraction as a whole and of most individual sugars was generally found during the first months of lactation.

6'-SL as well as LSTc declined in a similar manner, a fact that supports the hypothesis that a single sialyltransferase, possibly ST6GalI, exclusively accepting type 2 structures is involved in the biosynthesis of these carbohydrates. The more pronounced decrease of LSTc can be explained with a decrease of core LNnT, the precursor of LSTc (Table 4), whereas the amounts lactose, precursor of 6'-SL, remained constant after one week postpartum. 3'-SL decreasing to a small extent during the study period could be synthesized by ST3Gal IV or also by ST3Gal VI, two α2,3-sialytransferases acting preferentially on type 2 structures, and we found that LSTa, a minor acidic sugar, declined very much and could not or only partially detected after 2 and 3 months. We hypothesize that ST3Gal III, an α2,3-sialytransferase acting preferentially on type 1 structures, is involved in the biosynthesis of LSTa as well as of DSLNT. LSTb was the only acidic carbohydrate that increased within the first month postpartum confirming previous results. A so-called ST6GlcNAc could transfer sialic acid moieties to subterminal GlcNAc yielding LSTb as well as DSLNT, an oligosaccharide exhibiting both α2,6-linked and α2,3-linked neuraminic acid.

### Biological implications

Human milk oligosaccharides, especially those compounds containing lacto-N-biose (type 1 structure) or GlcNAc, are thought to promote growth of a favorable gut flora in newborns. The total core structures LNT and LNH, exhibiting lacto-N-biose units and representing the major GlcNAc-containing oligosaccharides in human milk, attain maximum levels after approximately 1 week postpartum and decline thereafter almost twofold until three months postpartum. Therefore it can be concluded that newborns obtain the best protection from human milk oligosaccharides during the first few weeks of lactation. In addition, we found out that secretor women corresponding to milk groups one and three, significantly produced higher amounts of these core structures than nonsecretors of milk group two with the potential consequence that the gut flora of nonsecretor mothers differs analogously with a lower occurrence of protective species, like Bifidobacteria, Lactobacilli.

Several in vitro studies but also a clinical study suggest the function of especially α1,2-fucoslyoligosaccharides to reduce infectious diseases, including diarrhea, by inhibiting adherence of pathogens to epidermal surfaces, because they function as soluble competitive inhibitors of the host cell surface ligand and pathogen receptor interaction. It was reported that babies fed with human milk of group three (Le(a-b-)), exhibiting a high proportion of α1,2-fucosyloligosaccharides, were significantly less infected by stable toxin-E. coli than babies fed with milk of Le(a-b+)-mothers (milk group one). It can be hypothesized that newborns fed with milk of nonsecretor mothers, lacking α1,2-fucosylated sugars and H-antigens, are even more susceptible to stable toxin-E.coli. In this context the relatively high frequencies of nonsecretors (se/se) in Europe (20%), as compared to the low occurrence of nonsecretors for example in the mestizo population of Mexico (1%) has to be discussed. Possibly the high incidence of secretors evolved among the Mexican population also as a result of this pathogenic E.coli species. In other regions with a different evolutionary pressure of pathogens, nonsecretors may be better apt to feed babies successfully resulting in a higher incidence of this Lewis blood group. Apart from the above mentioned H-antigens, human milk oligosaccharides exhibit a variety of other antigenic determinants, like Le^{a}, Le^{b}, Le^{x}, in dependence of the Lewis blood groups of the mothers as shown in Table 1.

### Example 2 Infant milk formula

An infant formula for feeding an infant who has Lewis blood type Le(a-/b+) or Le(a-/b-) or Le(x-/y+) or Le(x-/y-) comprising per 100 ml (66 kcal):
1.3 g protein (whey and casein)
7.3 g digestible carbohydrates (including lactose)
3.5 g fat (vegetable fat, fish oil)
1.0 g non-digestible oligosaccharides of which 200 mg 2'-fucosyllactose, 720 mg beta-galacto-oligosaccharides, and 80 mg fructo-oligosaccharides

Further are included: choline, myo-inositol, taurine, minerals, trace elements, and vitamins as known in the art

### Example 3 Toddler milk composition

Toddler milk composition (intended for infants 1-3 years) for feeding a toddler who has Lewis blood type Le(a-/b+) or Le(a-/b-) or Le(x-/y+) or Le(x-/y-) comprising per 100 ml (67 kcal; 15.1 dry weight):
1.5 g protein (whey protein/casein 1/1 w/w)
8.5 g digestible carbohydrates (of which 6.0 g lactose 1.1 g maltodextrin)
3.0 g fat (vegetable fats)
1.2 g non-digestible oligosaccharides of which 200 mg 2'-fucosyllactose 900 mg beta-galacto-oligosaccharides, and 100 mg fructo-oligosaccharides
Minerals, trace minerals, vitamins as known in the art including choline and taurine

### Example 4 Nutritional supplement

Nutritional supplement for supplementing the milk of a mother who has Lewis blood type Le(a+/b-) packed in a sachet with the following composition:
A:
   500 mg 2'-fucosyllactose
   1.5 g maltodextrin
   Advised dosage is 1 to 4 sachets of supplement A per 100 ml breast milk.
B:
   1 g 2'-fucosyllactose
   1 g maltodextrin
   Advised dosage is 1 or2 sachets of supplement A per 100 ml breast milk.

## Claims

1. Use of a composition comprising 2-fucosylated oligosaccharides for the preparation of a nutritional composition for feeding an infant, said infant having blood group A, B, or AB according to the ABO blood group system.

2. The use according to claim 1, wherein the nutritional composition is a nutritional supplement.

3. The use or according to claim 1, wherein the mother of said infant has has blood group O according to the ABO blood group system.

4. The use according to any one of claims 1-3, wherein the nutritional composition further comprises non-digestible oligosaccharides selected from fructooligosaccharides and galactooligosaccharides.

5. The use according to any one of claims 1-4, wherein the nutritional composition further comprises uronic acid oligosaccharides selected from the group consisting of galacturonic acid oligosaccharides and pectin degradation products.

6. The use according to any one of claims 1-5, wherein the nutritional composition fed to the infant comprises 100 mg to 2 g 2-fucosylated oligosaccharides per 100 ml.

7. Use according to any one of claims 1-6, wherein the composition comprises lipid that provides 35 to 50% of the total calories, protein that provides 7.5 to 12.5% of the total calories, and digestible carbohydrate that provides 40 to 55% of the total calories.

8. The use according to any one of claims 1-7, wherein the 2-fucosylated oligosaccharides comprise alpha-1,2-fucosyl oligosaccharides, preferably 2'-fucosyllactose and/or lacto-N-fucopentaose I.

9. Nutritional supplement comprising 2-fucosylated oligosaccharides for use in supplementing an infant formula or a human milk for feeding an infant, having blood group A, B, or AB according to the ABO blood group system.

10. Method for preparing a nutritional composition comprising supplementing milk from a mother that has blood group O according to the ABO blood group system.

11. The method according to claim 10, wherein the nutritional composition is for feeding an infant that has blood group A, B, or AB according to the ABO blood group system.
